Europäisches Patentamt

European Patent Office

Office européen des brevets

(11) Veröffentlichungsnummer: **0 513 485 A1**

# EUROPÄISCHE PATENTANMELDUNG

(21) Anmeldenummer: **92103545.7**

(22) Anmeldetag: **02.03.92**

(51) Int. Cl.⁵: **A61B 17/12, A44B 11/12**

(30) Priorität: **16.05.91 EP 91107941**

(43) Veröffentlichungstag der Anmeldung:
**19.11.92 Patentblatt 92/47**

(84) Benannte Vertragsstaaten:
**AT BE CH DE DK ES FR GB GR IT LI LU NL SE**

(71) Anmelder: **Pohl, Josef Peter, Dipl.-Ing. (FH)**
**38, Kreuzschanze**
**W-6500 Mainz 1(DE)**

(72) Erfinder: **Pohl, Josef Peter, Dipl.-Ing. (FH)**
**38, Kreuzschanze**
**W-6500 Mainz 1(DE)**

(74) Vertreter: **Dr. Fuchs, Dr. Luderschmidt**
**Dipl.-Phys. Seids, Dr. Mehler Patentanwälte**
**Abraham-Lincoln-Strasse 7, Postfach 4660**
**W-6200 Wiesbaden(DE)**

(54) Vorrichtung zum Abbinden von Gliedmassen (Venenstauer).

(57) Die Erfindung betrifft eine Vorrichtung zum Abschnüren von Gliedmaßen, einen sogenannten Venenstauer, die ein Abschnürband (2) aufweist, welches mit seinem einen Ende (6) mittels eines Steckers (8) lösbar in einem Bandschloß befestigbar ist, und dessen anderes Ende (13) unter Schlaufenbildung durch das Bandschloß zurückgeführt ist und in diesem mittels einer Klemmwippe (58) arretierbar ist, die bei Ausübung einer Zugkraft auf den Stecker (8) über ein Zwischenteil (72) automatisch betätigbar ist, welches die Klemmwippe (58) im hinteren Abschnitt (62) untergreift. Zum Aufheben der Klemmwirkung ist als separates Bauteil eine Drucktaste (18, 64) vorgesehen, die schwenkbar unter einem Quersteg (48) im Bandschloß abgestützt ist und mit einem Druckpunkt (68) auf dem hinteren Teil (62) der Klemmwippe aufliegt. Mittels einer Schiebetaste (20) läßt sich die Steckverbindung (76,78) lösen.

Der Venenstauer ist in jeder Hinsicht ergonomisch und mit hohem Bedienungskomfort ausgestattet.

FIG. 2

Die Erfindung betrifft eine Vorrichtung zum Abbinden von Gliedmaßen eines Menschen nach dem Oberbegriff des Patentanspruches 1. Solche Vorrichtungen werden allgemein auch als Venenstauer bezeichnet, da sie insbesondere dafür verwendet werden, eine Stauwirkung auf die Blutgefäße beispielsweise im Oberarm eines Patienten bei der Blutabnahme auszuüben.

Venenstauer bestehen üblicherweise aus einem Gummigurt bzw. aus einem Gummiband und einem Bandschloß, mit dem sich das eine Ende des Bandes lösbar verbinden läßt. Das andere Ende ist unter Bildung einer Schlaufe durch das Bandschloß hindurchgeführt, in dem es in unterschiedlichsten Positionen festgeklemmt werden kann. Bei Anziehen des anderen Bandendes durch das Bandschloß hindurch zur Verengung der Bandschlaufe um beispielsweise den Arm eines Patienten herum, erfolgt die Klemmung des Bandes im Bandschloß automatisch. Um eine Zugentlastung des unter Spannung stehenden Bandes zu bewirken, ist das Bandschloß im allgemeinen mit einer Drucktastenfläche versehen, bei deren Betätigung die Bandklemmung vorübergehend aufgehoben wird. Zum schnellen Abnehmen des Venenstauers vom Arm kann die lösbare Verbindung des ersten Bandendes mit dem Bandschloß gelöst werden.

Ein gattungsgemäßer Venenstauer ist aus der DE-A-2824037 bekannt. Bei diesem bekannten Venenstauer ist die Drucktastenfläche hinter dem hinteren Abschnitt der Klemmwippe am schlaufenseitigen Ende des Bandschlosses auf den Verbindungsmitteln für das eine Ende des Bandes mit dem Bandschloß angeordnet. Diese Lage der Drucktastenfläche ist für einen hohen Bedienungskomfort nicht besonders geeignet, außerdem besteht die Gefahr, daß bei Betätigen der Drucktastenfläche zum Lösen der Bandklemmung gleichzeitig auch die Verbindungsmittel für das Band unbeabsichtigt gelöst werden.

In der EP-A-0196646 ist zwar ein Venenstauer beschrieben, bei dem die Drucktastenfläche mehr in den vorderen Teil des Bandschloßes verlegt ist, die im Anspruch 1 dieser Druckschrift beschriebene Anordnung ist jedoch nicht funktionsfähig, weil sie ein Gelenkdreieck definiert, welches nach physikalischen Grundsätzen nicht beweglich ist. Eine funktionsfähige Lösung kann bestenfalls der Zeichnung dieser Druckschrift entnommen werden. Diese Lösung erfordert jedoch eine zusätzliche translatorische Verschiebbarkeit der Drehachse der Klemmwippe, durch die beim Klemmen und Lösen des Bandes ein höherer Bandverschleiß entsteht. Außerdem ist die Drucktastenfläche auch hier einstückig mit den Verbindungsmitteln für das eine Bandende ausgebildet.

Der Erfindung liegt die Aufgabe zugrunde, einen Venenstauer der gattungsgemäßen Art derart weiterzubilden, daß sein Bedienungskomfort verbessert wird, indem insbesondere eine Drucktastenfläche vorgesehen wird, die sich auch in den vorderen, der Bandschlaufe abgewandten Bereich des Bandschlosses erstreckt, wobei das Prinzip einer ausschließlich drehschwenkbaren Klemmwippe beibehalten und der Druck auf die Klemmwippe zum Lösen der Bandklemmung in einem im wesentlichen vorgegebenen Punkt des hinteren Klemmwippenabschnittes erfolgen soll.

In bevorzugter Ausgestaltung soll der erfindungsgemäße Venenstauer derart ausgebildet sein, daß das Bandschloß für das Zusammenfügen seiner Einzelteile keine zusätzlichen, insbesondere metallischen Verbindungsmittel erfordert und ohne mechanische Hilfsmittel aus seinen Einzelteilen zusammensetzbar ist.

Diese Aufgabe wird für einen gattungsgemäßen Venenstauer gemäß der DE-A-2824037 grundsätzlich durch die kennzeichnenden Merkmale des Patentanspruches 1 gelöst. Dadurch, daß für die Drucktastenfläche ein von den anderen Funktionselementen des Bandschlosses unabhängiges Bauteil vorgesehen ist, ergibt sich eine größere Gestaltungsfreiheit für die Bedienungsfunktionen des erfindungsgemäßen Venenstauers. Das Drucktastenbauteil muß so ausgebildet sein, daß seine Drucktastenfläche einerseits in bedienungsfreundlicher Posiiton betätigbar ist, und daß es möglichst in einem bestimmten Punkt mit festgelegtem Hebelarm auf den hinteren Abschnitt der Klemmwippe drückt, so daß deren Funktion unabhängig davon gewährleistet ist, wo sich die Drucktastenfläche im einzelnen am Bandschloß befindet.

Diese Bedingungen können gemäß den kennzeichnenden Merkmalen des Patentanspruches 1 grundsätzlich auch durch eine in Betätigungsrichtung parallel im Schloßgehäuse geführte Drucktaste erzielt werden. Eine solche Parallelführung erfordert aber, wenn sie verklemmungssicher arbeiten soll, im allgemeinen einen relativ hohen Konstruktionsraum, weswegen das Drucktastenbauteil gemäß der Erfindung vorzugsweise an einem seiner Enden schwenkbar gelagert ist. Bei einer solchen Ausführungsform kann der Hebelarm vom Schwenkpunkt zum Betätigungspunkt noch dazu beitragen, daß der Kraftaufwand zum Betätigen der Drucktaste gegenüber einer direkten Betätigung der Klemmwippe noch verkleinert wird.

Die in dieser Beschreibung verwendeten Begriffe "vorne" und "hinten" wie auch die Begriffe "oben" und "unten" beziehen sich auf die normale Handhabungsposition des Bandschlosses, in der das hintere Ende das bandschlaufenseitige Ende bezeichnen soll und das vordere Ende das der Bandschlaufe abgewandte Ende, wogegen die Oberseite diejenige ist, die die Drucktastenfläche aufweist.

In zwei wesentlichen Ausführungsformen der Erfindung ist das Drucktastenbauteil in einer Variante an seinem vorderen Ende schwenkbar im Schloßgehäuse gelagert, bei einer anderen Variante am hinteren Ende. Die Lagerung am vorderen Ende hat den Vorteil, daß der Druckpunkt, mit dem das Drucktastenbauteil auf dem hinteren Abschnitt der Klemmwippe aufliegt, praktisch ans hintere Ende des Drucktastenbauteils und gleichzeitig auch weitgehend an das hintere Ende des hinteren Abschnittes der Klemmwippe verlegt werden kann. Hierbei ergibt sich eine relativ günstige Hebelübersetzung, wenn man nicht gerade auf das vordere Ende der Drucktaste drückt.

Eine schwenkbare Lagerung des Drucktastenbauteils an seinem hinteren Ende läßt zwar nur einen kleinen Hebelarm von dem Schwenkpunkt des Drucktastenbauteils zum Druckpunkt auf die Klemmwippe zu, die Drucktaste kann aber über diesen Druckpunkt hinaus nach vorne frei auskragend verlängert werden, wobei sich eine sehr einfache Betätigung mit geringer Kraft an diesem verlängerten Ende im vorderen Bereich des Bandschlosses ergibt.

Die bauliche Trennung der Drucktaste beispielsweise von der Klemmwippe bietet die Möglichkeit zu einer besonders bevorzugten Ausführung der Erfindung, bei der nämlich die einseitige Lagerung des Drucktastenbauteiles derart ausgeführt ist, daß sich das Drucktastenbauteil in seinem Schwenklagerbereich zusätzlich translatorisch von der Schwenkachse weg in Richtung auf die Klemmwippe hin bewegen läßt. Erfolgt nämlich die Betätigung der Drucktastenfläche im Bereich der einseitigen Lagerung des Drucktastenbauteiles, ist ein Lösen der Klemmwippe dennoch möglich. Wird die Drucktastenfläche dagegen an dem der Schwenklagerung abgewandten Ende jenseits des Druckpunktes auf die Klemmwippe betätigt, wird das Drucktastenbauteil über den Druckpunkt auf die Klemmwippe als Abstützungspunkt am entgegengesetzten Ende nach oben in seine Lagerung gedrückt und dort abgestützt, die in diesem Fall den Hebeldrehpunkt bildet.

Die Lagerung der Drucktaste im Gehäuse kann auf unterschiedliche Art und Weise erfolgen. So ist es möglich, daß das Drucktastenbauteil seitliche Drehzapfen aufweist, die in Bohrungen oder Ausschnitte der seitlichen Gehäusewände eingreifen. Für eine Lagerung, die in Richtung auf die Klemmwippe hin zusätzlich auch noch eine translatorische Bewegung in begrenztem Rahmen zuläßt, ist es sinnvoll, die Lagerung durch am Ende des Drucktastenbauteiles vorgesehene Haken auszubilden, die einen Quersteg im Gehäuse nur teilweise von unten umfassen, so daß sie sich bei der oben beschriebenen Möglichkeit der Betätigung der Drucktaste in der Nähe der Lagerung von diesem Quersteg entfernen können.

Ist die Schwenkachse des Drucktastenbauteiles auf diese Art und Weise am hinteren Ende des Drucktastenbauteiles vorgesehen, kann es vorteilhaft sein, zwischen dem Drucktastenbauteil und dem hinteren Abschnitt der Klemmwippe ein Federelement vorzusehen, welches diese beiden Bauteile jeweils auseinandergespreizt hält. Dadurch wird einerseits erreicht, daß die Klemmwippe für einen freien Banddurchlauf immer im geöffneten Zustand gehalten wird, wenn sie nicht durch Zug in der Bandschlaufe in ihre Klemmposition gedrückt wird, wodurch allgemein der Bandverschleiß vermindert wird. Andererseits würde ein solches Federelement das Drucktastenbauteil bei der vorstehend beschriebenen hakenartigen Lagerung immer in seine an der Schwenkachse anliegende Lagerposition gedrückt halten.

Um das Drucktastenbauteil an einem zu weiten Herausschwenken aus dem Gehäuse zu hindern, kann es sinnvoll oder erforderlich sein, zusätzlich zu den Lagerabstützungen seitlich der Drucktastenfläche bestimmte Ansätze am Drucktastenbauteil vorzusehen, die die Kanten des Gehäuseausschnittes für die Drucktastenfläche untergreifen und so eine Bewegung der Drucktaste in Richtung aus dem Gehäuse hinaus begrenzen. Eine solche Begrenzung kann bei einseitig schwenkbar gelagerter Drucktaste natürlich auch dadurch erreicht werden, daß die Schwenklagerung mit einer Drehwinkelbegrenzung versehen ist.

Eine besondere Bedienungsfreundlichkeit des erfindungsgemäßen Venenstauers wird auch durch Merkmale der äußeren Gesamtgestaltung des Bandschlosses erreicht, dessen Gehäuse in bevorzugter Ausführungsform hinten mit einem schräg nach oben gerichteten Fortsatz versehen ist. In diesem Fortsatz ist der eine Teil der Verbindungsmittel für die lösbare Verbindung des einen Bandendes mit dem Bandschloß angeordnet, so daß dieses eine Bandende in das offene Ende des schrägen Fortsatzes unter Einrastung der Verbindungsmittel eingesteckt werden kann. Die Vorderseite des schräg nach oben gerichteten Gehäusefortsatzes bietet gleichzeitig in bedienungsfreundlicher Weise Platz für ein Betätigungsmittel zum Lösen der Rastverbindung des einen Bandendes und zum Auswerfen dieses Endes aus dem Bandschloß. Dieses Betätigungsmittel ist zweckmäßigerweise in Form einer Schiebetaste ausgeführt, die sich unmittelbar an die Drucktastenfläche anschließt und zum Auswerfen des Bandes leicht mit dem Daumen betätigbar bzw. nach oben schiebbar ist, wenn man das Bandschloß mit seiner Drucktastenfläche nach oben in der Hand hält.

Da die Rückführung der Bandschlaufe in das Bandschloß hinein und durch das Bandschloß hindurch in der Nähe der Unterseite des Gehäuses erfolgt, gewährleistet der schräge Gehäusefortsatz einen Abstand zwischen den in das Bandschloß eintretenden Enden der Bandschlaufe, wodurch ein eventuelles Einklemmen der Haut des Patienten zwischen diesen Schlaufenenden vermieden wird.

Da das lösbar mit dem Bandschloß verbundene eine Ende des Bandes mit den lösbaren Verbindungsmitteln in das Ende des schrägen Fortsatzes eintritt, wird ferner gewährleistet, daß dieses eine Ende, welches nach Verrastung mit dem Bandschloß in kraftschlüssiger Zugverbindung mit dem hinteren Abschnitt der Klemmwippe steht, durch die Schräge des Fortsatzes immer eine gleichgerichtete Zugkraft auf die Klemmwippe ausübt. Das Gehäuse des Bandschlosses ist vorzugsweise zweiteilig ausgeführt mit einem äußeren Gehäusemantel und einem Einschub, und zwar in einer Weise, daß die einzelnen Funktionselemente weitgehend im Einschub aber auch am Gehäusemantel zuerst angebracht werden, um dann die beiden Gehäuseteile zusammenzuschieben, die mittels eines Federelementes eine Rastverbindung mit einander eingehen und die einzelnen Funktionsteile im verbundenen Zustand im Gehäuse festlegen. Auf diese Weise ist eine Montage des Bandschlosses ohne technische Hilfsmittel möglich. Die einzelnen Funktionsteile sind ihrerseits derart ausgebildet, daß sie ohne zusätzliche Verbindungsmittel, wie Achsdrähte, Nieten und dergleichen in das Gehäuse einsetzbar sind, so daß sich alle Teile des Bandschlosses aus Kunststoff im Spritzgießverfahren herstellen lassen und so das gesamte Bandschloß nach beendeter Gebrauchstüchtigkeit nach Entfernen des Bandes einer einheitlichen Kunststoffwiederaufarbeitung zugeführt werden kann.

Das Fehlen metallischer Verbindungsmittel ist auch eine Voraussetzung dafür, daß die erfindungsgemäß Abschnürvorrichtung im Bereich der Magnet-Resonanz-Tomographie für röntgenologische Untersuchungen eingesetzt werden kann.

Die bevorzugte Gestaltung der Bauteile des Bandschlosses ergibt sich im einzelnen aus der Beschreibung der Ausführungsbeispiele.

Im folgenden werden zwei bevorzugte Ausführungsformen des erfindungsgemäßen Venenstauers unter Hinweis auf die beigefügten Zeichnungen näher erläutert.

Es stellen dar:

Figur 1
Eine seitliche Gesamtansicht eines Venenstauers,

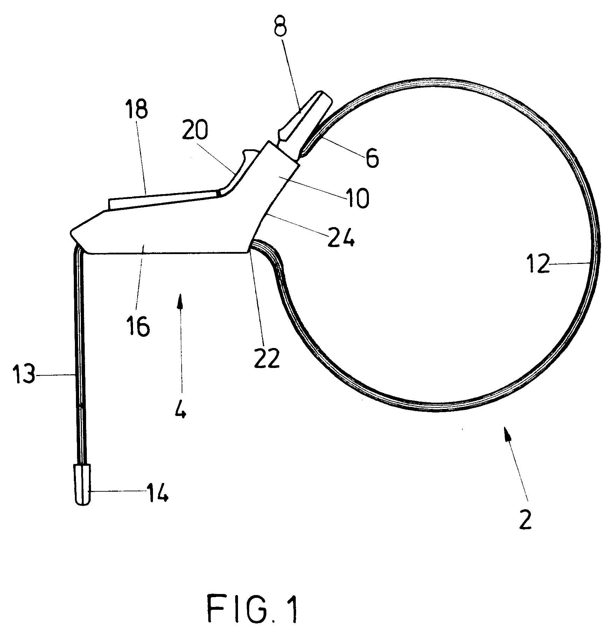

Figur 2
einen Längsschnitt durch das Bandschloß des Venenstauers nach Figur 1,

Figur 3
einen Längsschnitt durch das leere Gehäuse des Bandschlosses,

Figuren 4a bis c
einen Längsschnitt durch sowie eine Draufsicht auf den Gehäusemantel und eine Seitenansicht der in diesen einzusetzenden Schiebetaste,

Figuren 5a bis d
einen Längsschnitt durch und eine Draufsicht auf den Gehäuseeinschub sowie Seitenansicht der in diesen einzusetzenden Funktionsteile, nämlich der Klemmwippe, des Drucktastenbauteiles und des Zwischenteiles und

Figur 6
einen Längsschnitt durch eine abgewandelte Ausführungsform des Bandschlosses.

Der in Figur 1 als Ganzes in Seitenansicht dargestellte Venenstauer besteht im wesentlichen aus einem gummielastischen Abbindegurt oder Band 2, welches als ein Flachband ausgebildet ist, dessen Breitenerstreckung aus der Seitenansicht der Figur 1 nicht erkennbar ist, und einem Bandschloß 4. Das Band 2 ist an seinem einen Ende 6 mit einem Stecker 8 versehen, mit dem es im Wege einer weiter unten noch zu beschreibenden lösbaren Verbindung in das Ende eines schräg nach oben gerichteten Fortsatzes 10 des Bandschlosses 4 einsteckbar ist. Der Rest des Bandes 2 ist unter Bildung einer Schlaufe 12 in das Bandschloß 4 zurück und durch dieses hindurch geführt und ist an seinem anderen Ende 13 mit einem Stopper 14 zu sehen, der ein Hinausgleiten des Bandes 2 aus dem Bandschloß 4 verhindert.

Das Bandschloß 4 selbst weist ein Gehäuse 16 auf, an dessen Oberseite eine Drucktastenfläche 18 und eine Schiebetaste 20 zu erkennen sind.

Die Bedienungsweise des in Figur 1 dargestellten Venenstauers ist wie folgt:
Durch eine Schiebebewegung der Schiebetaste 20 nach oben wird der Stecker 8 aus dem Bandschloß in einer Weise gelöst, daß er aus dem Bandschloß 4 hinausspringt. Solange sich der Stecker 8 nicht im Bandschloß 4 befindet und keine Zugkraft auf ihn ausgeübt wird, ist das Band 2 durch das Bandschloß 4 hindurch frei beweglich. Durch Ziehen am gelösten Bandende 6 kann daher zum Vergrößern der für eine Schlaufe 12 benötigten Bandlänge das Band ungehindert hinten aus dem Bandschloß 4 herausgezogen werden, bis der Stopper 14 am Gehäuse 16 des Bandschlosses anschlägt. Das Band wird dann beispielsweise um den Arm eines Patienten gelegt und der Stecker 8 unter Selbstverrastung wieder in das Bandschloß 4 eingesteckt. Durch Ziehen am anderen Bandende 13 bzw. am Stopper 14 wird die gummielastische Bandschlaufe 12 um den Arm enger gezogen, wodurch auch eine Zugkraft des einen Bandendes 6 auf den Stecker 8 ausgeübt wird, durch die eine im Bandschloß angeordnete, weiter unten noch zu beschreibende Klemmvorrichtung betätigt wird, die ein Rückgleiten des Bandes durch das Bandschloß 4 hindurch in Richtung einer Vergrößerung der Bandschlaufe 12 verhindert. Durch Druck auf die Drucktastenfläche 18 kann die Klemmwirkung auf das Band 2 innerhalb des Bandschlosses 4 aufgehoben werden, so daß durch Drücken auf die Drucktastenfläche 18 eine Entlastung des Bandzuges erreicht werden kann. Um den Venenstauer schnell wieder vom Arm des Patienten abzunehmen, braucht lediglich wieder die Schiebetaste 20 betätigt zu werden, wodurch sich die Bandschlaufe 12 öffnet.

Aus Figur 1 sind bereits einige grundsätzliche Gestaltungsmerkmale des Venenstauers erkennbar. Durch den schrägen Gehäusefortsatz 10 wird ein Abstand zwischen dem mit dem Stecker 8 versehenen einen Bandende 6 und der Eintrittsstelle 22 der Bandschlaufe 12 in das Bandschloß 4 ermöglicht, der ein Einklemmen der Haut des Patienten zwischen diesen beiden Abschnitten des gummielastischen Bandes beim Verkleinern der Bandschlaufe 12 verhindert. Außerdem ist die Rückwand 24 des schrägen Fortsatzes 10 leicht gebogen ausgeführt, damit sie sich in Fortsetzung der Bandschlaufe 12 harmonisch an die Gliedmaße des Patienten anlegen kann.

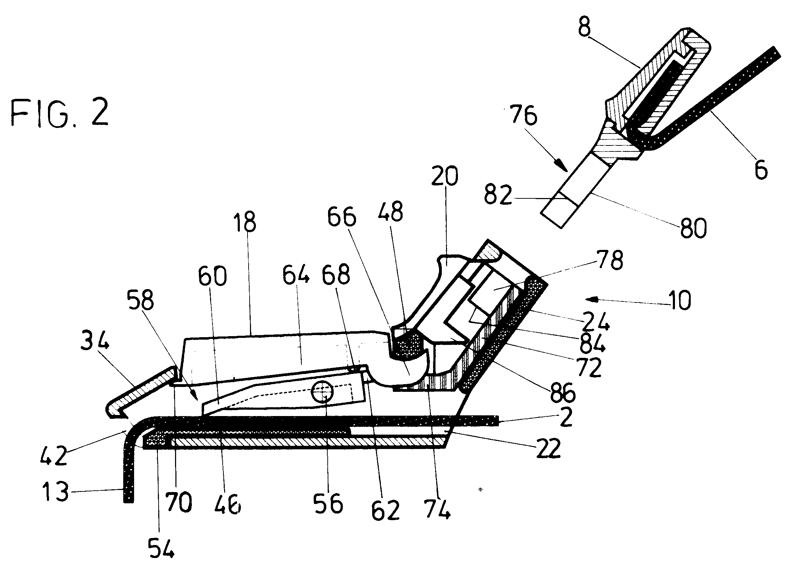

Gleichzeitig ist aus Figur 1 erkennbar, daß das Bandschloß 4, wenn man es mit Blick auf die Bandschlaufe von unten in der Hand hält, leicht zu betätigen ist, da sowohl die Drucktastenfläche 18 wie auch die Schiebetaste 20 aus der gleichen Handhaltung heraus mit dem Daumen leicht erreichbar sind. Im folgenden wird anhand der Figuren 2 bis 5 das Bandschloß 4 im einzelnen erläutert.

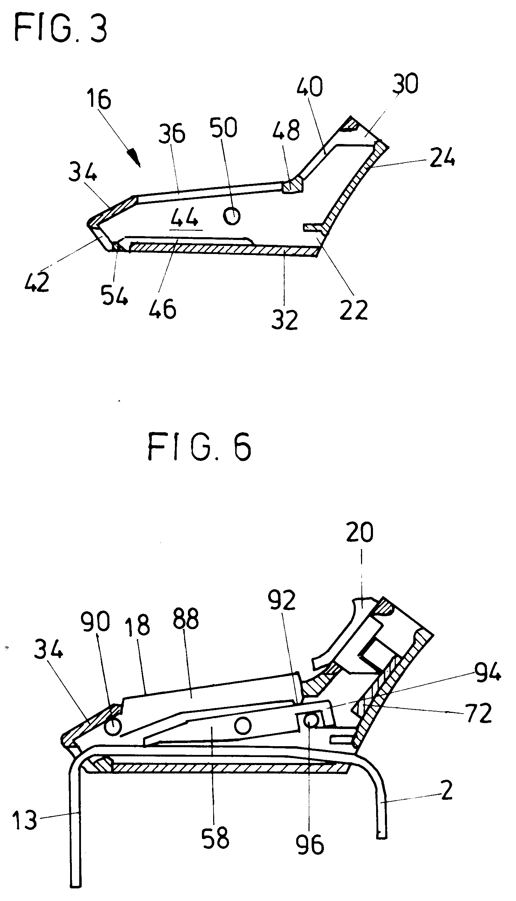

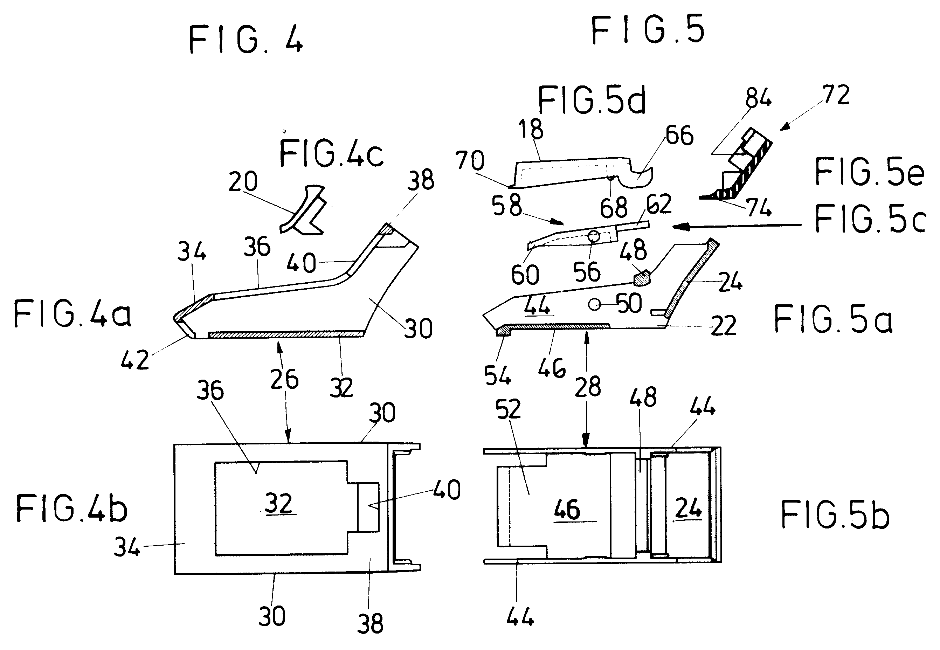

Figur 3 zeigt einen Längsschnitt durch das leere Gehäuse 16 des Bandschlosses, welches aus einem Außengehäuse 26 und einem Einschubteil 28 besteht. Das Außengehäuse 26 ist einzeln im Schnitt in Figur 4a und in Draufsicht in Figur 4b wiedergegeben, während das Einschubteil 28 im Längsschnitt in Figur 5a und in Draufsicht in Figur 5b dargestellt ist.

Bezug nehmend auf die Figuren 4a und 4b weist das nach hinten (in den Figuren rechts) offene Außengehäuse 26 neben zwei äußeren Seitenwänden 30, von denen in Figur 4a nur eine sichtbar ist, einen äußeren Boden 32, eine Oberwand 34 mit einem Drucktastenausschnitt 36 und eine Vorderwand 38 des schrägen Fortsatzes 10 auf, welche mit einem Ausschnitt 40 für die Schiebetaste 20 versehen ist. Am vorderen Ende (in der Figur links) ist das Außengehäuse noch mit einem vorderen Durchlaufschlitz 42 für das Band 2 versehen.

In ähnlicher Weise besteht das in den Figuren 5a und 5b dargestellte, nach oben im wesentlichen offene Einschubteil 28 aus zwei inneren Seitenwänden 44, einem inneren Boden 46, der Hinterwand 24 des schrägen Fortsatzes 10 und einem oberen Quersteg 48, der in zusammengesetztem Zustand des Gehäuses den Drucktastenausschnitt 36 von dem Ausschnitt 40 für die Schiebetaste trennt. Am unteren hinteren Ende des Einschubteiles ist der Eintrittschlitz 22 für das Band 2 vorgesehen, die inneren Seitenwände 44 sind je noch mit einer Lagerbohrung 50 versehen. An den inneren Boden 46 schließt sich vorne eine federnde Rastzunge 52 an, die an ihrer Vorderkante mit einer Rastkante 54 versehen ist, welche im zusammengesetzten Zustand des Gehäuses einrastend vor die Vorderkante des äußeren Bodens 32 des Außengehäuses 26 greift, wie dies aus Figur 3 zu erkennen ist.

Zur Unterscheidung des Außengehäuses und des Einschubteiles sind in Figur 3 die geschnittenen Teile des Außengehäuses von rechts oben nach links unten und die geschnittenen Teile des Einschubteiles von links oben nach rechts unten schraffiert. Gepunktet ist in Figur 3 die innere Seitenwand 44 des Einschubteiles angedeutet, auf die man in der Schnittdarstellung blickt.

Es sei nun auf Figur 2 Bezug genommen, in der das Gehäuse 16 in entsprechender Schnittdarstellung wie in Figur 3 erscheint, jedoch mit allen eingebauten Funktionselementen, die zusammen das Bandschloß 4 ergeben.

Mittels seitlicher Achszapfen 56 ist in den Lagerbohrungen 50 der inneren Seitenwände 44 eine Klemmwippe 58 drehbar gelagert, die einzeln nochmals in Figur 5c dargestellt ist. Wird diese Klemmwippe 58 entgegen dem Uhrzeigersinn um die Achszapfen 56 als Drehachse gedreht, klemmt sie mit ihrem vorderen Ende 60 das Band 2 gegen den inneren Boden 46. Da die Klemmwippe 58 an ihrem vorderen Ende 60 mit einer relativ spitzen Kante versehen ist, wird das Band 2 bei Einklemmung an einer Bewegung nach rechts, bezogen auf die zeichnerische Darstellung gehindert. Die Klemmwippe 58 weist rechts von ihrer Drehachse 56 einen hinteren Abschnitt 62 auf, der in Figur 2 zum Teil verdeckt ist, aber in Figur 5c in voller Länge zu erkennen ist. Als hinterer Abschnitt 62 soll der gesamte Bereich der Klemmwippe 58 bezeichnet sein, der sich rechts von der Drehachse 56 befindet.

Weiterhin Bezug nehmend auf Figur 2 ist oberhalb der Klemmwippe 58 ein Drucktastenbauteil 64 angeordnet, welches an seiner Oberseite mit der Drucktastenfläche 18 versehen ist. Das Drucktastenbauteil 64 weist an seinem hinteren, in der Figur 2 rechten Ende hakenartige Fortsätze 66 auf, mit denen es den Quersteg 48 des Einschubteiles 28 untergreift. Der Quersteg 48 stellt eine Schwenkachse dar, um die herum, geführt durch die hakenartigen Fortsätze 66 das Drucktastenbauteil 64 in begrenztem Rahmen verschwenkbar ist. Das Drucktastenbauteil 64 weist weiterhin einen Druckpunkt 68 auf, mit dem es auf dem hinteren Abschnitt 62 der Klemmwippe 58 aufliegt. Dieser Druckpunkt 68 ist im Ausführungsbeispiel als eine parallel zur Drehachse 56 der Wippe 58, d.h. senkrecht zur Zeichenebene sich erstreckende Druckkante ausgebildet. Schließlich ist das Drucktastenbauteil 64 an seiner Vorderseite, in der Zeichnung links, mit Fortsätzen 70 versehen, die die Oberwand 34 des Außengehäuses 26 hintergreifen und somit die Aufwärtsbewegung der Drucktaste aus dem Gehäuse hinaus begrenzen. Das Drucktastenbauteil 64 ist einzeln noch in Figur 5b dargestellt.

Als weiteres Bauteil ist im Gehäuse des Bandschlosses 4, und zwar in dessen schrägem Fortsatz 10 ein Zwischenteil 72 angeordnet, welches sich in Richtung dieses Fortsatzes 10 erstreckt und in dieser Richtung begrenzt in ihm in seiner Längsrichtung verschiebbar ist. Dieses Zwischenteil 72 untergreift mit einem unteren Fortsatz 74 den hinteren Abschnitt 62 der Klemmwippe 58 und übt somit bei einer Verschiebung im Fortsatz 10 nach oben eine Zugkraft auf den hinteren Abschnitt 62 der Klemmwippe 58 aus, die dadurch zu einer Drehung entgegen dem Urzeigersinn zur Bandklemmung veranlaßt wird. Das Zwischenteil 72, welches einzeln auch noch in Figur 5e dargestellt ist, dient ferner der lösbaren Verbindung mit dem Stecker 8 am einen Ende 6 des Bandes 2, um eine auf das Bandende 6 ausgeübte Zugkraft auf die Klemmwippe 58 zu übertragen.

Der Stecker 8 ist zu diesem Zweck mit Verbindungmitteln 76 versehen, welche mit Verbindungsmitteln 78 zusammenwirken, die am Zwischenteil 72 vorgesehen sind. Die Verbindungsmittel 76/78 erlauben eine einrastende Steckverbindung zwischen dem Stecker 8 und dem Zwischenteil 72. Die Verbindungsmittel 76 bestehen (was in der zeichnerischen Darstellung der Figur 2 im einzelnen nicht zu erkennen ist) im wesentlichen aus zwei elastisch spreizbaren Stiften 80, welche mit Widerhaken 82 versehen sind, die komplementäre Kanten 84 der Verbindungsmittel 78 am Zwischenteil 72 hintergreifen. Durch eine an der Schiebetaste 20 vorgesehenen Nase 86 lassen sich die eingerasteten Stifte 80 des Steckers 8 spreizen, um die Steckverbindung zu lösen.

Der Figur 2 ist einfach zu entnehmen, daß bei eingestecktem Stecker 8 und bei unter Zugkraft stehendem Bandende 6 die durch Linksdrehung in Klemmstellung gedrehte Klemmwippe 58 aus ihrer Klemmstellung entgegen der durch das Bandende 6 ausgeübten Zugkraft gelöst werden kann, indem durch Druck auf die Drucktastenfläche 18 über den Druckpunkt 68 der Klemmwippe 58 eine Rechtsdrehung erteilt wird, durch die das linke Ende 60 der Klemmwippe 58 gehoben und das Band freigegeben wird. Der an der Klemmwippe 58 unverändert wirksame Hebelarm zum Lösen der Klemmung ist der Abstand zwischen dem Druckpunkt 68 und der Drehachse 56 der Klemmwippe. Wird links vom Druckpunkt 68 auf die Drucktastenfläche 18 gedrückt, findet eine zusätzliche Kraftübersetzung statt, die sich aus dem Verhältnis des Abstandes des Betätigungspunktes zum Quersteg 48 zum Abstand zwischen dem Quersteg 48 und dem Druckpunkt 68 ergibt. Bei Betätigung der Drucktastenfläche 18 links vom Druckpunkt 68 stützt sich das Drucktastenbauteil in jedem Fall mit seinen hakenartigen Fortsätzen 66 unter dem Quersteg 48 ab. Es ist ferner leicht erkennbar, daß die Klemmwippe 58 auch bei einem Druck auf die Drucktastenfläche 18 oberhalb des Druckpunktes 68 oder sogar rechts davon gelöst werden kann, weil das Drucktastenbauteil in seiner Schwenklagerung um den Quersteg 48 herum nicht fixiert ist, sondern sich insgesamt nach unten drücken läßt. In der Regel wird die Drucktastenfläche 18 jedoch mehr zu ihrem vorderen Ende hin betätigt.

Im folgenden soll anhand der Figuren 4 und 5 die einfache Montage des Bandschlosses 4 erläutert werden.

In das Außengehäuse 26 (Figuren 4a und 4b) wird lediglich die Schiebetaste 20 vom Drucktastenausschnitt 32 her in den Schiebetastenausschnitt 40 von unten nach oben eingeführt, dessen seitliche Kanten sie mit (in der Zeichnung nicht erkennbar) entsprechenden seitlichen Ansätzen hintergreift. In das Einschubteil 28 (Figuren 5a und 5b) wird durch Spreizen der inneren Seitenwände 44 die Klemmwippe 58 (Figur 5c) mit ihren seitlichen Achszahpfen 56 in die Lagerbohrungen 50 eingeführt. Sodann wird das Zwischenteil 72 (Figur 5e) so hinter dem Quersteg 48 in das Einschubteil 28 eingesetzt, daß sein unterer Fortsatz 74 den hinteren Abschnitt 62 der Klemmwippe 58 untergreift. Schließlich wird von links her das Drucktastenbauteil 64 (Figur 5d) mit seinen hakenartigen Fortsätzen 66 unter den Quersteg 48 des Einschubteiles 28 geführt. Das so fertig bestückte Einzugteil 28 wird dann, auf die zeichnerische Darstellung bezogen, von rechts her in das Außengehäuse 26 eingeschoben, bis die Rastkante 54 des Einschubteiles hinter die Vorderkante des äußeren Bodens 32 des Außengehäuses einschnappt. Dadurch, daß die Seitenwände sowohl des Außengehäuses wie auch des Einschubteiles von vorne nach hinten in ihrer Höhe zunehmen, beide Teile also leicht konisch ausgeführt sind, ist bei Zusammensetzen beider Teile die Drucktastenfläche 18 ohne weiteres in ihren Ausschnitt 36 einführbar. Der Quersteg 48 bildet im montierten Zustand eine Trennung zwischen dem Ausschnitt 36 für das Drucktastenbauteil 64 und die Schiebetaste 20. Zum Auseinandernehmen des Bandschlosses braucht lediglich die Rastkante 54 des Einschubteiles von der Bodenseite des Schlosses her nach oben gedrückt zu werden, um das Einschubteil 28 wieder nach rechts aus dem Außengehäuse 26 herausziehen zu können. Zur Fertigmontage des gesamten Venenstauers wird das bereits mit dem Stecker 8 versehene Band mit seinem anderen Ende durch den Eintrittsschlitz 22 und den Austrittsschlitz 42 hindurch durch das Bandschloß 4 gezogen und dann am anderen Ende mit dem Stopper 14 versehen. Die Bandeinführung kann auch umgekehrt erfolgen.

In Figur 6 ist eine weitere Ausführungsform eines Bandschlosses dargestellt. Dieses Bandschloß unterscheidet sich von demjenigen gemäß Figur 2 im wesentlichen nur durch eine andere Ausführung des Drucktastenbauteiles und eine leicht geänderte Ausführung der Klemmwippe 58 und des Zwischenteiles 72 im Bereich von deren gegenseitiger Verhakung. Die Gehäuseteile stimmen mit der Ausführungsform nach der Figur 2 im wesentlichen überein.

Figur 6 zeigt ein Drucktastenbauteil 88, welches mit seinem vorderen Ende unterhalb der Oberwand 34 des Außengehäuses mittels zweier Achszapfen 90 schwenkbar gelagert ist. Aus Gründen der einfachen Montage ruhen die Achszapfen 90 in nach oben offenen Ausschnitten in den inneren Seitenwänden des Einschubteiles. Im montierten Zustand werden die Achszapfen 90 durch die sie überdeckende Oberwand 34 des Außengehäuses in ihren Lagerausschnitten gehalten. Der Druckpunkt 92 des Drucktastenbauteiles 88 befindet sich an dessen äußerstem hinteren Ende, wodurch ein verhältnismäßig großer Hebelarm an der Klemmwippe 58 erhalten wird. Dadurch wird zum Teil die etwas höhere Auslösekraft kompensiert, die bei dieser Ausführungsform erforderlich ist, wenn man verhältnismäßig weit vorne auf die Drucktastenfläche 18 drückt. Da sich die Schwenkzapfen 90 des Drucktastenbauteils 88 jedoch unterhalb des vorderen Teiles der Oberwand des Gehäuses befinden, ist bei Druck auf jede Stelle der Drucktastenfläche 18 eine Auslösung möglich.

Bei der Ausführungsform der Figur 6 ist der hintere Abschnitt der Klemmwippe 58 mit Haken 94 versehen, die von seitlichen Zapfen 96 des Zwischenteiles 72 untergriffen werden. Diese Ausführung ist mit derjenigen nach Figur 2 wirkungsgleich.

**Patentansprüche**

1. Vorrichtung zum Abbinden von Gliedmaßen, insbesondere des Oberarmes eines Patienten, mit
   - einem Band (2) dessen eines Ende (6) über Verbindungsmittel (8, 76, 78) lösbar mit dem hinteren Ende
   - eines Bandschlosses (4) verbindbar ist, und dessen anderes Ende (13) unter Bildung einer Schlaufe (12) durch das Bandschloß (4) zurückgeführt ist, in welchem das Band (2) in beliebigen Positionen festklemmbar ist, bei der
   - das Bandschloß (4) ein Gehäuse (16) mit einem Gehäuseboden (46) aufweist, in dem eine Bandklemmwippe (58) in etwa in ihrem mittleren Bereich ausschließlich drehbar (56) gelagert ist, deren vorderes, der Bandschlaufe (12) abgewandtes Ende (60) durch eine Schwenkbewegung um die drehbare Lagerung (56) gegen den Gehäuseboden (46) bewegbar ist, um das rückgeführte Band (2) zwischen sich und dem Gehäuseboden (46) zu klemmen, und deren hinter der Drehachse (56) liegende hinterer Abschnitt (62) über diese lösbaren Verbindungsmittel (76, 78) kraftschlüssig mit dem einen Teil (78) dieser Verbindungsmittel in Verbindung steht, und
   - das Bandschloß (4) eine durch einen Ausschnitt (36) in der Oberseite (34) seines Gehäuses (16) zugängliche, gegebenenfalls vorstehende Drucktastenfläche (18) aufweist, durch deren Betätigung die Bandklemmenwippe (58) entgegen dieser Schwenkbewegung zum Lösen der Bandklemmung betätigbar ist,

   dadurch gekennzeichnet, daß die Drucktastenfläche (18) Bereich eines von diesen Verbindungsmitteln (8, 76, 78) unabhängigen Drucktastenbauteils (64, 68) ist, welches außerhalb der Begrenzung des Ausschnittes (36) für die Drucktastenfläche (18) innerhalb des Gehäuses (16) seine Aufwärtsbewegung begrenzend abgestützt ist und mit einem Druckpunkt (68, 92) frei auf dem hinteren Abschnitt (62) der Klemmwippe (58) aufliegt.

2. Vorrichtung nach Anspruch 1, dadurch gekennzeichnet, daß das Drucktastenbauteil (88) mit seinem vorderen Ende mittels zweier seitlichen Zapfen (90) als Schwenkachse schwenkbar im Gehäuse (16) gelagert und der Druckpunkt (12) im Bereich des hinteren Endes des Drucktastenbauteils (88) vorgesehen ist.

3. Vorrichtung nach Anspruch 2, dadurch gekennzeichnet, daß das Drucktastenbauteil (88) seitlich der Drucktastenfläche (18) mit Vorsprüngen versehen ist, die die seitlichen Kanten des Ausschnitts (36) für die Drucktastenfläche (18) untergreifen.

4. Vorrichtung nach Anspruch 1, dadurch gekennzeichnet, daß die Abstützung (48, 66) des Drucktastenbauteils 64 im Bereich von dessen hinterem Ende vorgesehen ist, der Druckpunkt (68) sich zwischen dieser Abstützung (48, 66) und der Drehachse (56) der Klemmwippe (58) befindet und das Drucktastenbauteil (64) zur Ausbildung dieser Drucktastenfläche (18) über den Druckpunkt (68) auskragend nach vorne verlängert ist.

5. Vorrichtung nach mindestens einem der Ansprüche 1 bis 4, dadurch gekennzeichnet, daß das Gehäuse (16) an seinem hinteren Ende mit einem schräg nach oben gerichteten Fortsatz (10) versehen ist, in dem ein Zwischenteil (72) in Richtung dieses Fortsatzes (10) verschiebbar angeordnet ist, welches der kraftschlüssigen Verbindung zwischen dem hinteren Abschnitt (62) der Klemmwippe (58) und diesen Verbindungsmitteln (76, 78) dient und dafür einerseits den hinteren Abschnitt (62) der Klemmwippe (58) in einer Zugverhakung (74, 96) hintergreift und andererseits mit dem einen Teil (78) dieser Verbindungsmittel versehen ist.

6. Vorrichtung nach Anspruch 5, dadurch gekennzeichnet, daß das eine Ende (6) des Bandes (2) mit dem anderen Teil (76) dieser Verbindungsmittel versehen ist, welcher einrastbare Stifte (80) aufweist, die mit komplementären Gegenelementen (84) des einen Teils (78) dieser Verbindungsmittel verrastbar sind.

7. Vorrichtung nach Anspruch 5 oder 6, dadurch gekennzeichnet, daß der schräg verlaufende Gehäusefortsatz (10) mit einem Betätigungsmittel (20) zum Lösen der Verbindungsmittel (76, 78) versehen ist.

8. Vorrichtung nach mindestens einem der Ansprüche 1 bis 7, dadurch gekennzeichnet, daß das Gehäuse (16) zweiteilig ausgebildet ist, mit einem an der Hinterseite offenen Außengehäuse (26) mit einer äußeren Bodenwand (32) äußeren Seitenwänden (30), einer Oberwand (34) mit Ausschnitt für die Drucktastenfläche (18), einer Vorderwand (38) des schrägen Fortsatzes (10) und einem vorderen Schlitz (42) für das Band (2), und einem von hinten in das Außengehäuse (26) einschiebbaen und in diesem verrastbaren Einschub (28) mit inneren Seitenwänden (44) in denen zur Ausbildung dieser Drehachse (56) die Klemmwippe (58) mit seitlichen Achszapfen (56) drehbar gelagert ist, diesem Gehäuseboden (46), einer Hinterwand (24) des schrägen Fortsatzes (10), einem hinteren Schlitz (22) für das Band (2) und einem die inneren Seitenwände (44) verbindenden Quersteg (48) hinter dem hinteren Ende des Ausschnittes (36) für die Drucktastenfläche (18) im Außengehäuse (26).

9. Vorrichtung nach Anspruch 8, dadurch gekennzeichnet, daß die Seitenwände (30, 44) vom vorderen Ende des Gehäuses (16) aus nach hinten in ihrer Höhe zunehmen.

10. Vorrichtung nach Anspruch 8 oder 9, direkt oder indirekt zurückbezogen auf Anspruch 2 oder 3, dadurch gekennzeichnet, daß die seitlichen Achszapfen (90) der Schwenkachse des Drucktastenbauteils (88) in zwei nach oben offenen Einkerbungen in den inneren Seitenwänden (44) des Einschubteiles (28) ruhen, und daß die seitlichen Zapfen (90) bei in das Außengehäuse (26) eingesetzem Einschubteil (28) durch vordere obere Wandbereiche (34) des Außengehäuses (26) in ihren Einkerbungen gehalten sind.

11. Vorrichtung nach Anspruch 8 oder 9, direkt oder indirekt zurückbezogen auf Anspruch 4, dadurch gekennzeichnet, daß die Abstützung des Drucktastenbauteils (64) durch an dessen hinterem Ende vorgesehene Haken (66) gebildet ist, die den Quersteg (48) am Einschubteil (28) untergreifen, und daß im hinteren Abschnitt der Klemmwippe (58) erforderlichenfalls Ausschnitte zur Aufnahme dieser Haken (66) vorgesehen sind.

12. Vorrichtung nach Anspruch 11, dadurch gekennzeichnet, daß die Haken (66) sowohl eine Schwenkbewegung des Drucktastenbauteils (64) um den Quersteg (48) als Schwenkachse als auch eine begrenzte Abwärtsbewegung gegenüber dem Quersteg (48) zulassen.

13. Vorrichtung nach Anspruch 11 oder 12, dadurch gekennzeichnet, daß das Drucktastenbauteil, (64) an seinem vorderen Ende mit Anschlägen (70) versehen ist, die die vordere Kante des Ausschnittes (36) für die Drucktastenfläche (18) in Gehäuse (16) untergreifen.

14. Vorrichtung nach Anspruch 8 oder 9, direkt oder indirekt zurückbezogen auf Anspruch 4, dadurch gekennzeichnet, daß das Drucktastenbauteil an seinem hinteren Ende mittels seitlicher Zapfen in den Seitenwänden des Gehäuses schwenkbar gelagert ist.

15. Vorrichtung nach mindestens einem der Ansprüche 4 bis 9 und/oder 11 bis 14, dadurch gekennzeichnet, daß zwischen Drucktastenbauteil (64) und hinterem Abschnitt (62) der Klemmwippe (58) ein Druckfederelement vorgesehen ist.

16. Vorrichtung nach mindestens einem der Ansprüche 1 bis 15, dadurch gekennzeichnet, daß das Bandschloß (4), der Stecker (8) und der Stopper (14) ausschließlich aus Kunststoffteilen bestehten.

FIG. 1

FIG. 2

FIG. 3

FIG. 6

# FIG. 4

## FIG.4c

## FIG.4a

## FIG.4b

# FIG.5

## FIG.5d

## FIG.5e

## FIG.5c

## FIG.5a

## FIG.5b

EP 0 513 485 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | DE-A-3 104 382 (BAUMANN) <br> * Seite 9, Zeile 30 - Seite 10, Zeile 5 * <br> --- | 1 | A61B17/12 <br> A44B11/12 |
| A | DE-A-3 538 583 (BAUMANN) <br> * Zusammenfassung; Abbildung 1 * <br> --- | 1 | |
| A | GB-A-1 249 809 (MILLS) <br> * Abbildung 3 * <br> --- | 1 | |
| A | DE-U-9 006 941 (HOLTSCH) <br> * Abbildungen 1,2 * <br> ----- | 1 | |
| | | | **RECHERCHIERTE SACHGEBIETE (Int. Cl.5)** <br><br> A61B <br> A44B |

Der vorliegende Recherchenbericht wurde für alle Patentansprüche erstellt

| Recherchenort | Abschlußdatum der Recherche | Prüfer |
|---|---|---|
| DEN HAAG | 20 AUGUST 1992 | BARTON S. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
........................................................................
& : Mitglied der gleichen Patentfamilie, übereinstimmendes
    Dokument

EPO FORM 1503 03.82 (P0403)